# EUROPEAN PATENT SPECIFICATION

(11) **EP 1 614 443 B1**
(45) Date of publication and mention of the grant of the patent: **19.09.2007**
(21) Application number: 05254207.3
(22) Date of filing: 05.07.2005
(51) Int. Cl.: A61N 1/36

(54) **Brain implant device**
Gehirnimplantat
Dispositif d'implant de cerveau

(30) Priority: 07.07.2004 US 586368; 01.07.2005 US 173863
(43) Date of publication of application: 11.01.2006
(73) Proprietor: The Alfred E Mann Foundation for Scientific Research, Santa Clarita, CA 91380-9005 (US)
(72) Inventor: Schulman, Joseph H., Santa Clarita, CA 91387 (US)
(74) Representative: Carter, Stephen John

(56) References cited:
- WO-A-02/073526
- WO-A-20/04052456
- US-A- 4 785 827
- US-A- 4 969 468
- US-A- 5 215 088
- US-A1- 2004 088 024
- US-B1- 6 358 281

## Description

### FIELD OF THE INVENTION

This invention relates to an integrated, implantable apparatus for brain sensing and stimulation that wirelessly controls body functions.

### BACKGROUND OF THE INVENTION

Information is transmitted in the human body by the nervous system, which correlates and integrates various bodily processes, reactions and adjustments. Electrical pulses travel along the extension (axon) of a nerve cell, from one nerve cell to another, establishing functional pathways to make up the nervous system of the body. Thus, a function of a nerve is to take electric signals from various sources to the receiving locations within the body.

An electrode array may be used anywhere in a nervous system, at the end organs, (for example, without limitation, brain, kidney, liver, stomach, muscle or other tissue) or along the nerve (afferent or efferent) pathways in between. Sensory nerves in the body provide information as to the various bodily conditions, processes, reactions and adjustments. Such information is in the form of electrical signals and may be monitored by neurosensing using appropriate electrical or electronic equipment.

Certain nerves in the body direct muscular action and by electrically stimulating an appropriate nerve (neurostimulation), muscular action can be effected. In some cases, the muscle as well as other organs or excitable tissue may be directly or indirectly stimulated, in order to treat a disorder. Such treatment may include providing a number of coordinated, stimulation signals to various parts of the muscle or the body. "Stimulation" may mean providing signals which cause motion or movement of the muscles. "Stimulation" in connection with the brain may result in a physical response or it may result in a sensory response (such as vision, feeling, smell, etc.) which involves the senses rather than a physical motion. "Tissue" means any body tissue, nerve, muscle, organ, including the brain and the spinal cord, or other body part. "Excitable tissue" means tissue to which may be sent electrical signals in order to evoke a response, including a resulting beneficial effect on or treatment of the patient.

Both sensing and stimulating require a connection to transfer the electrical signal, whether it be nerve, muscle, organ, or otherwise. For example, there are usually thousands of fibers in a nerve, and each fiber may be carrying a unique signal. Consequently, problems arise as to how to connect to the desired location on, or within, the selected nerve, in order to sense signals from, or provide signals to, individual fibers.

Current techniques for connecting to nerves also involve nerve cuffs, which are placed around the nerve, and which can only stimulate large numbers of fibers within the nerve. It is known that multiple needle-electrodes may be individually inserted into the brain to make contact in a localized area.

In 1989, Byers, et al. (U.S. Patent No. 4,837,049) disclosed a very small implantable, biocompatible electrode array "bed of nails" having numerous small, sharp, conductive protuberances (needles) which penetrate brain, nerves, organs, muscle, or other body parts for electrical signal sensing or simulation. It is disclosed that the protuberances are carried on a rigid or flexible base and include electrical conductors connecting the protuberances to terminals for other electrical connections. An array placed on the brain can communicate wirelessly to control a muscle, nerve, or other body part. One or more arrays attached to the motor cortex of the brain, can transmit, in tetherless fashion, many channels of information to receiving body parts, such as muscles. This patent is incorporated herein by reference in its entirety.

In 1990, Byers, et al. (U.S. Patent No. 4,969,468) disclosed an implantable, biocompatible "bed of nails" electrode array for making multiple electrical contacts to electrically sense or stimulate biological tissues. The arrays may be used singly or in combination with a second array and may involve transmission, multiplexing, filtering, data processing or other electronic techniques. One or more electrode array attached to the motor cortex of the brain, in tetherless fashion, can transmit many channels of information to receiving body parts, such as muscles, to which electrode arrays are attached.

If the electrode array is to be used for sensing low voltage body signals, it is disclosed that an amplifier would be the first electrical circuit connected to the device. The signals may then be handled by analog or digital electronic methods. If the electrode array is to be used for electrically stimulating tissue, the terminals would be connected to circuits that provide to provide stimulation signals output to conductors which carry these stimulation signals from the terminals to the tissue-contacting needles. This patent is incorporated herein by reference in its entirety.

In 1993, Norman, et al. (U.S. Patent No. 5,215,088) disclosed an electrode array device for use as a neuron interface or as a cortical implant. A plurality of spire-shaped electrodes on a rigid base is disclosed. The electrodes are electrically isolated from each other at the base and may be multiplexed such that only a small number of lead wires need to be attached. The disclosed device may be used for a neuron interface and for a cortical implant for a vision prosthesis. This patent is incorporated herein by reference in its entirety.

WO 2004/052456 describes a modular implantable medical device that includes a plurality of separately housed and flexibly interconnected modules. One of the modules includes control electronics within a housing, wich is formed of a rigid material such as titanium, stainless steel or ceramic. Conductors extend from hermetic feedthroughs within the housing to lead connector modules for leads that can connect the control module to remotely disposed electrodes.

The complete package that is implantable in the skull to stimulate and or sense signals from the brain has not previously been disclosed. A need exists for an integrated, biocompatible, implantable brain implant device to effect body function.

### SUMMARY OF INVENTION

The invention provides a motor cortex stimulator as set forth in claim 1.

### BRIEF DESCRIPTION OF THE DRAWINGS

**FIG. 1** illustrates a cross-sectional view of the stimulator.

**FIG. 2** depicts a bottom view of the stimulator.

**FIG. 3** is a perspective view of the stimulator and attached electrode assembly.

### DETAILED DESCRIPTION OF THE PREFERRED EMBODIMENT

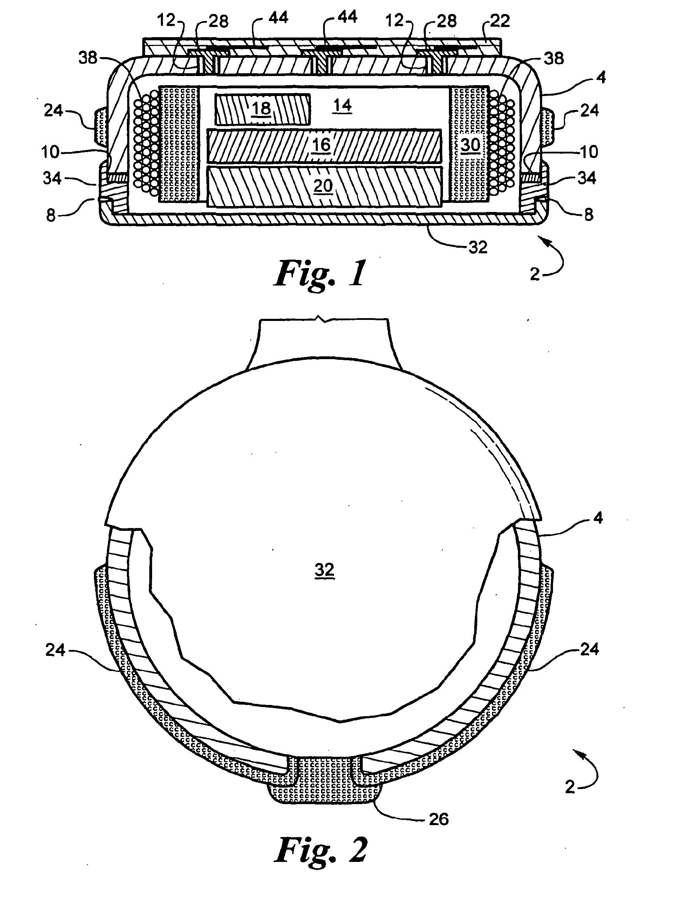

**FIG. 1** presents a cross-sectional view of the implantable sensor 2 comprising a ceramic case 4 that is comprised of a biocompatible material that is selected from the group of ceramic materials, such as alumina, titania, zirconia, stabilized-zirconia, partially-stabilized zirconia, tetragonal zirconia, magnesia-stabilized zirconia, ceria-stabilized zirconia, yttria-stabilized zirconia, and calcia-stabilized zirconia, sapphire, and in a preferred embodiment ceramic case 4 is yttria-stabilized zirconia. In an alternative embodiment the ceramic case 4 is comprised of tetragonal zirconia. The case 4 is preferably a round circular cylinder with an integral enclosure on one end and an opening on the other having dimensions of about 0.20 to 0.24 inches high and about 0.8 inches in outer diameter with a wall thickness of about 0.010 inches.

The ceramic case 4 forms a part of a hermetic enclosure for various processing electronic components 14 including, but not limited to, capacitors and oscillator crystal 16, chip stack 18, and battery 20. These components process the electrical signals that are generated by the brain and are wirelessly transmitted via antenna 24 to remote location or locations in the body to effect function in the targeted living tissue.

The battery charging system comprises the battery 20 that is charged by a remotely transmitted charging signal that is in turn detected by a coil 38 and that is processed in part by a ferrite 30.

The case 4 contains a plurality of feedthroughs 12, numbering as many as about 128, or 64 in a preferred embodiment, plus at least about two indifferent electrodes (not illustrated), where each feedthrough 12 is associated with an electrode 28 which conducts the electrical signals that are generated by the brain through the wall of the case 4 and to the processing electronics 14. In a preferred embodiment, the diameter of each feedthrough 12 is about 0.010 to 0.020 inches. Each electrode 28 is hermetically sealed by known techniques to the case 4 at the interface formed by the electrode 28 and the feedthrough 12. The electrodes 28 each have a rivet-like appearance with an outer diameter of the external button portion of about 0.030 inches.

An important aspect of the invention is that the case 4 is closed by hermetically attaching a biocompatible interface ring 34 by brazing and then a diaphragm 32 by biocompatible means, preferably laser welding, to assure a hermetic seal. An interface ring 34 is placed between the preferably round case 4 and the diaphragm 32 to enable the joint to be formed. A braze ring 10 is located between the case 4 and the interface ring 34 to effect a braze joint between the diaphragm 32, interface ring 34, and the case 4 upon thermal processing. The braze ring 10 is comprised of a material that is selected from the class of known braze alloys for bonding case 4, which is preferably comprised of ceramic, to interface ring 34. The braze ring 10 is preferably selected from the group of biocompatible braze materials, such as 50%Ti-50%Ni, substantially pure nickel, or 33%Ti-67%Ni, percentages expressed in weight percent.

The interface ring 34 is comprised of Ti-6Al-4V or Ti-8Al-1Mo-1V. The Ti-8Al-1Mo-1V has a relatively high electrical resistance of about 120 times that of copper, while the electrical resistance of Ti-6Al-4V is about 100 times that of copper. In a preferred embodiment, the interface ring 34 is comprised of Ti-8Al-1Mo-1V to minimize antenna 24 loses of efficiency. In a preferred embodiment, the ring has a cross sectional thickness of about 0.002 inches and is about 0.100 inches high, spanning the space between the case 4 and the diaphragm weldment 8.

Preferably, the diaphragm 32 is comprised of a biocompatible weldable material, preferably Ti-6Al-4V. In a preferred embodiment, the diaphragm 32 is welded, preferably by laser welding, although electron beam or other welding process are applicable, to interface ring 34, thereby creating weldment 8. It is important that the braze joint to case 4, that is created before welding of the diaphragm 32, not be adversely affected by the later applied welding process, hence the thin cross-section of interface ring 34 which limits heat transfer to the braze joint during the welding process. In addition, in a preferred embodiment, the distance between the braze ring 10 and the weldment is about 0.1 inches, which helps avoid braze joint thermal effects from the welding operation.

**FIG. 2** presents a bottom view of the sensor 2, showing the antenna 24 and diaphragm 32. Antenna 24 is located on the outer surface of case 4 and is connected to the electronics 14, that are located inside case 4, by a passage through the wall of case 4. A biocompatible, electrically insulating coating 26 is placed over the antenna by known ceramic deposition processes. The coating 26 is preferably comprised of alumina, although it may also be a non-ceramic, such as an epoxy.

Antenna 24 is preferably a painted or silk screen applied conductor that is formed by applying an electrically conductive metal in powder from paste suspension. The metal is silver. Once applied, the dried antenna 24 is heated under controlled conditions to remove the organic binders and result in an electrically conductive antenna 24. One known silver paste is silver epoxy 6144S, supplied by Lord Corporation, North Carolina, United States of America.

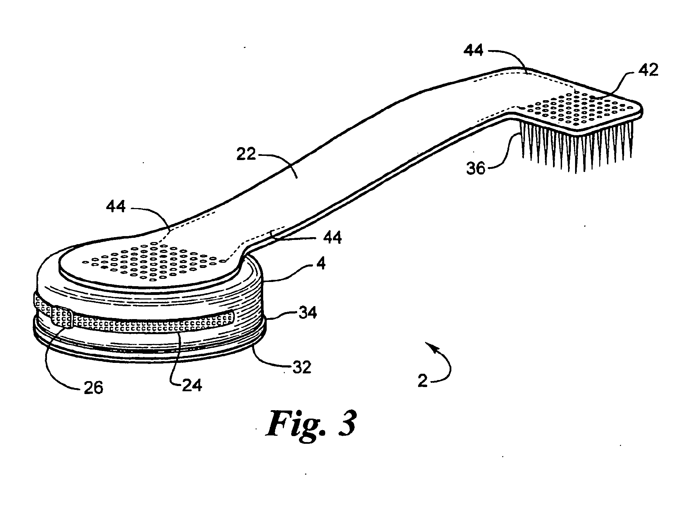

The sensor 2 and integrally attached flex connector 22 that connects to neuro-needle array 42 are illustrated in FIG. 3. The neuro-needle array 42 preferably has an approximately square matrix of 8 by 8 neuro-needles 36 totaling 64 in number, although the array 42 may contain as many as about 128 needles. The array 24 is preferably described by the 1990, Byers, et al. U.S. Patent No. 4,969,468 to a biocompatible "bed of nails" electrode array for making multiple electrical contacts to electrically sense or stimulate biological tissues, which is incorporated by reference herein in its entirety. In a preferred embodiment the needles are comprised of a biocompatible electrically conductive metal, such as platinum.

The flex connector 22 is comprised of an electrically insulating and biocompatible material that contains a plurality of electrical conductors 44. Each electrical conductor 44 is attached to a neuro-needle 36 and to an electrode 28 to conduct electrical signals therebetween. The electrical conductor 44 is bonded by known methods, such as gold bump bonding, to electrode 28 (**FIG. 1**). A preferred form of conductor 44 is a biocompatible wire, such as gold wire. The matrix of the flex connector 22 is preferably comprised of polyimide, such as Kapton®, or amorphous fluoropolymers, such as Teflon®, or silicone are also candidates. A bonding layer 21, preferably of epoxy, is formed to attach the flex connector 22 to ceramic case 4 and to electrodes 28.

Thus, in accordance with this invention, by utilizing a completely implantable sensor 2, it is now possible to control the movement of a muscle by normal brain function when the normal nerve path is severed or otherwise not functioning. This is a surprising result since implantable self-contained brain generated signal receiver/processors have not been previously disclosed.

Obviously, many modifications and variations of the present invention are possible in light of the above teachings. It is therefore to be understood that, within the scope of the appended claims, the invention may be practiced otherwise than as specifically described.

## Claims

1. A motor cortex stimulator or sensor (2) comprising:
a ceramic case (4) having a wall with an outside surface that encloses electronic components (14); and
at least one electrically conductive feedthrough (12) in the wall of said ceramic case (4);
**characterised in that:**
said electrically conductive feedthrough (12) contains an electrode (28); and
the stimulator or sensor (2) comprises an antenna (24), the product of thermal processing of silver paste, bonded to the outside surface of the wall of said ceramic case (4).

2. The motor cortex stimulator or sensor (2) according to claim 1, wherein said motor cortex stimulator is biocompatible.

3. The motor cortex stimulator or sensor (2) according to claim 1 or claim 2 further comprising a metal interface ring (34), wherein said metal interface ring (34) is brazed to said ceramic case (4).

4. The motor cortex stimulator or sensor (2) according to claim 3 further comprising a metal lid (32), wherein said metal lid (32) is laser welded to said metal interface ring (34).

5. The motor cortex stimulator or sensor (2) according to any one of the preceding claims, wherein said at least one electrically conductive feedthrough (12) comprises sixty-four feedthroughs (12).

6. The motor cortex stimulator or sensor (2) according to any one of the preceding claims, further comprising:
an array (42) of neuro-needles (36) each attached to an electrical conductor (44); and
a flex connector (22) containing said electrical conductor (44), wherein said electrical conductor (44) is attached to said electrode (28).

## Patentansprüche

1. Stimulator oder Sensor für den motorischen Cortex (2), umfassend:
ein Keramikgehäuse (4), das eine Wand mit einer Außenoberfläche aufweist, die elektronische Komponenten (14) umschließt; und
zumindest ein elektrisch leitfähiges Hindurchführungsmittel (12) in der Wand des Keramikgehäuses (4);
**dadurch gekennzeichnet, dass** das elektrisch leitfähige Hindurchführungsmittel (12) eine Elektrode (28) beinhaltet,
und
der Stimulator oder Sensor (2) eine Antenne (24) umfasst, die ein durch Wärmebehandlung von Silberpaste erhaltenes Produkt ist, das an die Außenoberfläche der Wand des Keramikgehäuses (4) angehaftet ist.

2. Stimulator oder Sensor für den motorischen Cortex (2) nach Anspruch 1, wobei der Stimulator für den motorischen Cortex biologisch verträglich ist.

3. Stimulator oder Sensor für den motorischen Cortex (2) nach Anspruch 1 oder Anspruch 2, der ferner einen Metallübergangsring (34) umfasst, wobei der Metallübergangsring (34) an das Keramikgehäuse (4) gelötet ist.

4. Stimulator oder Sensor für den motorischen Cortex (2) nach Anspruch 3, der ferner einen Metalldeckel (32) umfasst, wobei der Metalldeckel (32) an den Metallübergangsring (34) lasergeschweißt ist.

5. Stimulator oder Sensor für den motorischen Cortex (2) nach einem der vorangegangenen Ansprüche, worin das zumindest eine elektrisch leitfähige Hindurchführungsmittel (12) vierundsechzig Hindurchführungsmittel (12) umfasst.

6. Stimulator oder Sensor für den motorischen Cortex (2) nach einem der vorangegangenen Ansprüche, ferner umfassend:
eine Anordnung (42) von Neuro-Nadeln (36), die jeweils an einem elektrischen Leiter (44) angebracht sind; und
einen Flex-Verbinder (22), der den elektrischen Leiter (44) enthält, wobei der elektrische Leiter (44) an der Elektrode (28) angebracht ist.

## Revendications

1. Stimulateur ou capteur de zone motrice (2) comprenant :
un boîtier céramique (4) ayant une paroi avec une surface extérieure qui renferme des composants électroniques (14) ; et
au moins une sortie électriquement conductrice (12) dans la paroi dudit boîtier céramique (4) ;
**caractérisé en ce que** :
ladite sortie électriquement conductrice (12) contient une électrode (28) ; et
le stimulateur ou capteur (2) comprend une antenne (24), le produit d'un traitement thermique d'une pâte d'argent, liée à la surface extérieure de la paroi dudit boîtier céramique (4).

2. Stimulateur ou capteur de zone motrice (2) selon la revendication 1, où ledit stimulateur de zone motrice est biocompatible.

3. Stimulateur ou capteur de zone motrice (2) selon la revendication 1 ou la revendication 2, comprenant en outre une bague d'interface métallique (34), où ladite bague d'interface métallique (34) est brasée audit boîtier céramique (4).

4. Stimulateur ou capteur de zone motrice (2) selon la revendication 3, comprenant en outre un couvercle métallique (32), où ledit couvercle métallique (32) est soudé par laser à ladite bague d'interface métallique (34).

5. Stimulateur ou capteur de zone motrice (2) selon l'une des revendications précédentes, où au moins une sortie électriquement conductrice précitée (12) comprend soixante-quatre sorties (12).

6. Stimulateur ou capteur de zone motrice (2) selon l'une des revendications précédentes, comprenant en outre :
une rangée (42) de neuro-aiguilles (36) chacune fixée à un conducteur électrique (44) ; et
un connecteur flexible (22) contenant ledit conducteur électrique (44), où ledit conducteur électrique (44) est fixé à ladite électrode (28).
